# EUROPÄISCHE PATENTSCHRIFT

(11) **EP 4 232 591 B1**
(45) Veröffentlichungstag und Bekanntmachung des Hinweises auf die Patenterteilung: **18.06.2025**
(21) Anmeldenummer: 22809361.3
(22) Anmeldetag: 21.10.2022
(51) Int. Cl.: C12P 5/02, C12M 1/107, C12C 11/00, C12C 13/00, C12F 3/06, C12M 1/00, C12M 1/33, A23J 3/34, C12P 21/06, C05F 5/00, C12P 7/06, A23J 1/14, C05F 17/40, C05F 17/50

(54) **VERFAHREN ZUM BETREIBEN EINER BRAUEREI UND BRAUEREI MIT BIOGASGEWINNUNG**
BREWERY OPERATING METHOD AND BREWERY WITH BIOGAS RECOVERY
PROCÉDÉ POUR FAIRE FONCTIONNER UNE BRASSERIE ET BRASSERIE AVEC PRODUCTION DE BIOGAZ

(30) Priorität: 22.10.2021 DE 102021127533; 18.07.2022 DE 102022117862
(43) Veröffentlichungstag der Anmeldung: 30.08.2023
(73) Patentinhaber: Steinecker GmbH, 85356 Freising-Attaching (DE)
(72) Erfinder: SCHNEID, Ralph, 85356 Freising (DE); SCHEIDEL, Alexander, 85356 Freising (DE)
(74) Vertreter: Grünecker Patent- und Rechtsanwälte PartG mbB
(86) Internationale Anmeldenummer: PCT/EP2022/079420
(87) Internationale Veröffentlichungsnummer: WO 2023/067162

(56) Entgegenhaltungen:
- DE-A1- 102008 053 543
- DE-A1- 102016 014 103
- DE-A1- 102018 009 592
- CALISKAN GULIZAR ET AL: "Anaerobic Biodegradation of Beer Production Wastewater at a Field Scale and Explotation of Bioenergy Potential of Other Solid Wastes from Beer Production", INTERNATIONAL JOURNAL OF RENEWABLE ENERGY AND BIOFUELS, vol. 2014, 664594, 30 March 2014 (2014-03-30), pages 1 - 15, XP093147962, ISSN: 2333-0589, DOI: 10.5171/2014.664594
- ARANTES MABEL KARINA ET AL: "Treatment of brewery wastewater and its use for biological production of methane and hydrogen", INTERNATIONAL JOURNAL OF HYDROGEN ENERGY, vol. 42, no. 42, 22 September 2017 (2017-09-22), pages 26243 - 26256, XP085239758, ISSN: 0360-3199, DOI: 10.1016/J.IJHYDENE.2017.08.206
- PANJICKO MARIO ET AL: "Biogas production from brewery spent grain as a mono-substrate in a two-stage process composed of solid-state anaerobic digestion and granular biomass reactors", JOURNAL OF CLEANER PRODUCTION, ELSEVIER, AMSTERDAM, NL, vol. 166, 17 August 2017 (2017-08-17), pages 519 - 529, XP085197355, ISSN: 0959-6526, DOI: 10.1016/J.JCLEPRO.2017.07.197

## Beschreibung

Die Erfindung betrifft ein Verfahren zum Betreiben einer Brauerei mit Biogasgewinnung und eine Brauerei mit einer Brauanlage, einer Biomassekonversionsanlage und einem Blockheizkraftwerk.

Bierbrauanlagen benötigen bekanntermaßen vergleichsweise großen Menge an thermischer und elektrischer Energie für Heiz-, Koch- und Kühlprozesse bzw. den Betrieb der hierfür verwendeten Anlagen. Eine Vielzahl qualitätsbestimmender Parameter hängt zudem von den für die einzelnen Prozesse eingesetzten Energiemengen ab, sodass sich diese allein durch Prozessoptimierung nur begrenzt reduzieren lassen. Daher versucht man, den fossilen Energiebedarf zu senken und durch regenerative Quellen abzudecken, ohne dabei die Produktqualität zu verschlechtern.

Die wichtigsten Reststoffe, welche bei Brauprozessen anfallen, sind Treber, (Alt-)Hefe, Malzstaub und Abwasser. Alle zeichnen sich durch einen relativ hohen Gehalt an biochemischen Substanzen aus, welche eine energetische Verwertung prinzipiell ermöglichen. Hierfür sind Biogasanlagen bekannt, um aus diesen Reststoffen methanhaltiges Biogas zu gewinnen. Dieses wird gereinigt und üblicherweise Erdgas beigemischt und in einem Heizkessel oder Blockheizkraftwerk verfeuert. Die Beimischung ist notwendig, da herkömmliche Biogasanlagen entweder keine bedarfsgerechte Energiemenge erzeugen können oder für eine entsprechend umfassende Verwertung der Reststoffe so groß dimensioniert werden müssten, dass ein wirtschaftlicher Betrieb nicht gegeben wäre. Dies liegt hauptsächlich an den Trebern, welche den größten Anteil der Reststoffe ausmachen und unter anderem wegen eines hohen Gehalts an Lignin von Mikroorganismen relativ ineffizient umgesetzt werden. Da die Fermentationsdauer für eine ausreichende Umsetzung mehrere Tage betragen würde, wird anfallender Treber aus wirtschaftlichen Gründen oft nicht oder nur in vergleichsweise geringen Anteilen zur Biogaserzeugung verwendet und meist nichtregenerativ entsorgt. Somit geht prinzipiell wiederverwertbare Energie in erheblichem Ausmaß verloren.

Aus der DE 10 2016 014 103 B4 ist eine modulare Biomassekonversion bekannt, bei der biologische Reststoffe wie beispielsweise Abwässer oder Klärschlämme zuerst durch Dispergieren und Proteolyse zur Abscheidung eines Protein-Hydrolysats und dann durch Versäuerung und Abscheidung eines Mineraldüngers vorbehandelt werden, um schließlich Biogas und gereinigtes Abwasser mit gegenüber einstufigen Prozessen gesteigerter Rückgewinnungsrate zu erhalten.

Zwar können herkömmliche Biogasanlagen von Brauanlagen einen relativ hohen Methangehalt von 70 bis 80 % erreichen im Vergleich zu den ansonsten üblichen 60 bis 65 %. Dies ist jedoch nur bei meist ausschließlicher Verwertung von Abwasser möglich, da dieses in der Regel einen für die Umsetzung vorteilhaft hohen pH-Wert aufweist sowie einen hohen Kohlehydratanteil.

Zudem ist aus der DE 10 2014 001 907 A1 ein Verfahren zur stofflichen und energetischen Verwertung biogener Reststoffe von Braustätten durch Einsatz der Reststoffe in einer Nassfermentation bekannt. Dabei stellt man zum einen bei der Fermentation gewonnenes entschwefeltes Biogas für eine energetische Verwertung bereit und konzentriert zum anderen das gesamte Nährstoffpotential in Form von Stickstoff, Phosphor, Kalium und Schwefel in einer aus den Fermentationsrückständen gewonnenen Düngemittelfraktion. Hierfür werden die Reststoffe zuerst suspendiert und dann aerob hydrolysiert. Das dabei gewonnene Hydrolysat wird dem Biogas erzeugenden Fermentersystem zugeführt. Allerdings ist die so erzielbare Biogasausbeute unbefriedigend. Auch wird eine überwiegend energetische Verwertung der in den Reststoffen enthaltenen Nährstoffe zunehmend als unzulässig angesehen, da dies beispielsweise gegen Vorgaben der EU-Kreislaufwirtschaftsverordnung verstößt.

Die DE 10 2018 009592 A1 beschreibt ferner ein Verfahren zur Optimierung der Biologischen Reinigung von kommunalem und industriellem Abwasser hinsichtlich der resultierenden Abwasserqualität und einer umfassenden Verwertung von Inhaltsstoffen unter Einbeziehung der aus der DE 10 2016 014 103 B4 bekannten Biomassekonversion. Demnach kann der Kohlenstoffgehalt des Abwassers durch Zugabe einer größeren Menge Bioreststoff, wie beispielsweise Treber aus einer Brauerei, aufgestockt und aus dem Proteinanteil der Biomasse durch enzymatische Hydrolyse eine Nährstofflösung mit Aminosäuren und Peptiden gewonnen werden. Nach Aufarbeitung des Überschussschlamms mit Versäuerung wird in einer Fermentationsstufe aus der Säurelösung und Prozesswasserüberschuss Biogas gewonnen mit einem Methangehalt von etwa 57 %. Aus dem Biogas können in einem Blockheizkraftwerk Strom und Wärme gewonnen werden, die zur Deckung des Energiebedarfs der Abwasserreinigung herangezogen werden.

Es besteht demgegenüber somit weiterer Verbesserungsbedarf für einen möglichst energiesparenden Betrieb von Brauanlagen unter möglichst umfassender Verwertung aller dort anfallender Reststoffe mit möglichst geringem apparativen Aufwand und/oder unter flexibler Anpassung an einen betriebsabhängig schwankenden Energiebedarf.

Die gestellte Aufgabe wird mit einem Verfahren nach Anspruch 1 und einer Brauerei nach Anspruch 13 gelöst. Vorteilhafte Ausführungsformen sind in den Unteransprüchen angegeben. Das Verfahren dient zum Betreiben einer Brauerei und umfasst eine in Konverterstufen modular steuerbare Biomassekonversion von Braureststoffen umfassend Treber und alkalisches kohlenhydrathaltiges Abwasser. Bei der Biomassekonversion behandelt man wenigstens den Treber durch Proteolyse unter Abscheidung von Protein-Hydrolysat in einer ersten Konverterstufe und durch Versäuerung, und insbesondere Ionenaustausch, unter Abscheidung von Mineraldüngemittel in einer zweiten Konverterstufe vor und gewinnt darauf basierend durch Biogasfermentation Biogas in einer dritten Konverterstufe, unter gleichzeitiger Aufbereitung des Abwassers beispielsweise zu Prozesswasser. Ferner verfeuert man das gewonnene Biogas wenigstens anteilig zur Strom- und/oder Wärmeversorgung der Brauerei.

Dadurch kann im Treber und im Abwasser enthaltene Biomasse gemeinsam verarbeitet und mit unerwartet hoher Methanausbeute verwertet werden.

Die Vorbehandlung der Braureststoffe, insbesondere die Abtrennung von Mineraldüngemittel vor der Biogasfermentation, verbessert die Umsetzungsrate der Mikroorganismen, sodass die Biogaserzeugung wesentlich wirtschaftlicher in Anschaffung und Betrieb ist als für Brauereien bekannt. Es hat sich diesbezüglich überraschend herausgestellt, dass mit der stufenweisen Biomassekonversion auch bei mengenmäßig überwiegender Verwertung von Treber in Kombination mit Abwasser Methangehalte von bis zu über 90 % im Biogas erreicht werden. Die Abtrennung von Mineraldüngemittel als Vorbehandlung in Kombination mit dem alkalischen und relativ kohlehydrathaltigen Abwasser führt demnach zu einer unerwarteten Erhöhung des Methangehalts.

Dadurch zusätzlich gewonnenes Methan trägt zum energieautarken Brauanlagebetrieb bei und kann im Falle eines Gasüberschusses gespeichert oder anderweitig genutzt oder wirtschaftlich verwertet werden. Somit kann der nominelle Bedarf einer Brauanlage an thermischer und elektrischer Energie regenerativ gedeckt und der Bedarf für externe Energieversorgung minimiert werden. Anders gesagt ist ein im Wesentlichen energieautarker Brauanlagebetrieb möglich. Somit ist eine gleichermaßen kostengünstige und nachhaltige Produktion gegeben.

Zudem sind auch die abgeschiedenen Wertstoffe, also Protein-Hydrolysat und/oder eine optional gewonnene, beispielsweise zuvor ausfiltrierte Proteinfraktion, als Rohstoff zur Herstellung von Lebensmitteln wirtschaftlich verwertbar, ebenso abgeschiedenes Mineraldüngemittel zur Verwendung in der Landwirtschaft.

Die Biomassekonversion erfolgt in einer modular aufgebauten Biomassekonversionsanlage mit seriell geschalteten und separat steuerbaren Konverterstufen. Die zugeführten Braureststoffe werden somit in getrennt voneinander maschinell steuerbaren und entsprechend auch aufeinander abstimmbaren Prozessstufen behandelt. Darunter ist zu verstehen, dass beispielsweise die relativen Mengen bei der Vorbehandlung abgeschiedener Wertstoffe, wie Protein-Fraktionen, Protein-Hydrolysat und/oder Mineraldüngemittel, mit der relativen Menge des durch Biogasfermentation produzierten Biogases beispielsweise je nach Zusammensetzung der Braureststoffe und/oder Biogasbedarf untereinander abgestimmt werden können.

Vorteilhaft an der stufenweisen Biomassekonversion ist gerade für Brauereien, dass feste und flüssige Braureststoffe in einer gemeinsamen Anlage verarbeitet und verwertet werden können. Treber muss in herkömmlichen Biogasanlagen in der Regel getrennt von Abwasser behandelt werden. Dies wird erfindungsgemäß durch die beschriebene Vorbehandlung von Treber vermieden, wonach in der Biomassekonversionsanlage jeweils verbliebene Feststoffpartikel in flüssige Phasen eingeleitet und mit dem Brauanlagenabwasser, also Abwasser aus Brauprozessen oder aus anderen Prozessen in der Brauerei vermischt werden können, wodurch sich von herkömmlicher Biogaserzeugung bekannte Ausbeuteverluste vermeiden lassen.

Die Biomassekonversion der Braureststoffe kann einen Zellaufschluss in der ersten und/oder zweiten Konverterstufe umfassen. Ein solcher Zellaufschluss kann die Effektivität des beschriebenen Verfahrens steigern, wird wegen des dafür nötigen, relativ großen apparativen Aufwands beispielsweise abhängig von wirtschaftlichen Überlegungen gegebenenfalls optional integriert. Der Zellaufschluss erfolgt dann vorrangig in der ersten Konverterstufe, ist prinzipiell aber auch in der zweiten Konverterstufe möglich.

Die Biomassekonversion der Braureststoffe erfordert aber nicht zwingend einen Zellaufschluss in der ersten und/oder zweiten Konverterstufe. Beispielsweise kann eine mechanische Zerkleinerung (welche noch nicht zwangsläufig einen Zellaufschluss bewirken muss) der Braureststoffe ausreichend sein.

Das Protein-Hydrolysat kann durch Abtrennung, die beispielsweise eine Separation und insbesondere Tellerseparation oder beispielsweise eine Membranfiltration und insbesondere Ultrafiltration ist, abgeschieden werden. Ergänzend oder alternativ kann eine das Mineraldüngemittel enthaltende Nähr-und Düngerlösung durch anaerobe Versäuerung und anschließende Abtrennung, die beispielsweise eine Separation und insbesondere Tellerseparation oder beispielsweise eine Membranfiltration und insbesondere Ultrafiltration ist, und durch nachgeschalteten Ionenaustausch abgeschieden werden. Eine derartige Abscheidung ist besonders effizient und erleichtert die Weiterverarbeitung der damit erzeugten Fraktionen.

Die Biomassekonversionsanlage kann hierfür ferner umfassen: eine der ersten Konverterstufe zugeordnete Trenneinheit zur Abscheidung des Protein-Hydrolysats mittels Separation, insbesondere Zentrifugalseparation, oder zur Membranfiltration, insbesondere Ultrafiltration; und/oder eine der zweiten Konverterstufe zugeordnete Trenneinheit zur Separation, insbesondere Zentrifugalseparation, oder zur Membranfiltration, insbesondere Ultrafiltration, mit nachgeschaltetem Ionentauscher zur Abscheidung einer das Mineraldüngemittel enthaltenden Nähr-und Düngerlösung.

Um die Reinheit des abgeschiedenen Protein-Hydrolysats (umfasst Aminosäuren und Peptide) zu erhöhen, ist ein zusätzlicher Verfahrensschritt zu dessen Aufreinigung möglich, beispielsweise durch Proteinfällung mit anschließender Abtrennung der ausgefallenen Peptide und/oder Aminosäuren mittels Filtration und/oder Zentrifugalseparation. Ergänzend oder alternativ ist die Aufreinigung des Protein-Hydrolysats durch Chromatographie möglich.

Entsprechend kann die Biomassekonversionsanlage zur zusätzlichen Aufreinigung des (abgeschiedenen) Protein-Hydrolysats wenigstens eine der ersten Konverterstufe zugeordnete Aufreinigungseinheit umfassen, beispielsweise: eine Einheit zur Proteinfällung und anschließenden Filtration und/oder Zentrifugalseparation ausgefallener Peptide und/oder Aminosäuren; und/oder eine Einheit zur chromatographischen Trennung (Chromatographie-Einheit).

Die obige zusätzliche Aufreinigung des Protein-Hydrolysats ist mit allen beschriebenen und/oder dargestellten Ausführungsformen des Verfahrens und der Biomassekonversionsanlage möglich und ist daher als generelle Option der Protein-Hydrolysat-Abscheidung zu verstehen, auch wo dies nicht explizit erwähnt ist.

Vorzugsweise speichert man aus der ersten Konverterstufe in die zweite Konverterstufe zu leitendes Retentat (erzeugt aus den Braureststoffen) in wenigstens einem Puffergefäß zwischen, insbesondere bei diskontinuierlichem Betrieb der ersten Konverterstufe und kontinuierlichem Betrieb der zweiten Konverterstufe. Optional erfolgt auch eine Zwischenspeicherung von Medium / Sauerwasser (erzeugt aus dem Retentat) zwischen der zweiten und dritten Konverterstufe, wobei letztere dann vorzugsweise ebenso kontinuierlich betrieben wird. Somit kann die erste Konverterstufe diskontinuierlich je nach Anfallen / Verfügbarkeit der Braureststoffe arbeiten, also im sogenannten Batch-Betrieb, während in der zweiten und dritten Konverterstufe ein für Mikroorganismen und darauf beruhende biochemische Prozesse günstiger im Wesentlichen durchgehender Betrieb möglich ist.

Vorzugsweise wird ein erster Anteil des gewonnenen Biogases im Bereich der Brauerei zwischengespeichert, und/oder man speist einen zweiten Anteil des Biogases in ein externes Gasnetz ein. Der erste und/oder zweite Anteil des Biogases kann durch maschinelle Steuerung, also beispielsweise mittels wenigstens eines elektronisch gesteuerten Gasverteilers, der Zwischenspeicherung bzw. externen Verwendung zugeführt werden.

Durch die stufenweise Biomassenkonversion kann Methan aufgrund der Gesamtenergiebilanz von Brauprozessen prinzipiell im Überschuss erzeugt werden. Überschüssiges Biogas / Methan kann vor Ort gespeichert werden und beispielsweise zum Anfahren von Brauprozessen bzw. zugehörigen Anlagenteilen nach einer Revisionspause verwendet werden. Wird überschüssiges Biogas über einen längeren Zeitraum nicht verwendet, kann es aufgrund der Flüchtigkeit von Methan vorteilhaft sein, Biogas in ein externes Gasnetz einzuspeisen und dadurch wirtschaftlich zu verwerten. Auch könnte überschüssiges Biogas zur Versorgung anderer Produktionsanlagen genutzt werden, wie für die Limonadenherstellung, beispielsweise falls dort keine oder weniger entsprechend verwertbare Reststoffe anfallen

Vorzugsweise verfeuert man einen dritten Anteil des gewonnenen Biogases in einem Blockheizkraftwerk und/oder einen vierten Anteil des Biogases in einem Heizkessel zur Wärmeerzeugung (in Form von Dampf oder Heißwasser), beispielsweise für die Warmwassererzeugung (zum Beispiel für Einmaischwasser, Anschwänzwasser und/oder Spülwasser) oder für die Heißwassererzeugung als Heizwasser (zum Beispiel für die Beheizung von Wärmeverbrauchern wie beispielsweise einer Maischevorrichtung). Dadurch lässt sich die im Biogas enthaltene Energie je nach Wärme- und Strombedarf der Brauanlage flexibel und effizient in Wärme und elektrischen Strom umsetzen. Der dritte und/oder vierte Anteil des Biogases kann durch maschinelle Steuerung, also beispielsweise mittels wenigstens eines elektronisch gesteuerten Gasverteilers, dem Blockheizkraftwerk bzw. dem Heizkessel zugeführt werden.

Vorzugsweise führt man den mit dem dritten Anteil des Biogases erzeugten Strom einem anlageninternen Stromverbraucher, insbesondere der Brauanlage, und/oder einem anlageninternen Ladungsspeicher zu, und/oder man speist den Strom anteilig in ein externes Stromnetz ein.

Aufgrund der gesteigerten Effektivität der Biogaserzeugung kann das Blockheizkraftwerk aus den Braureststoffen mehr Strom produzieren als die Brauanlage selbst benötigt. Solche Überkapazitäten könnten durch Speicherung im Bereich der Brauerei gepuffert und/oder in Abstimmung mit einem Stromnetzbetreiber gezielt zur Lastregulierung genutzt werden. Beispielsweise ist auch ein damit abgestimmter Betrieb von Ladesäulen für Elektrofahrzeuge denkbar.

Vorzugsweise wird aus dem dritten und/oder vierten Anteil des Biogases erzeugte Wärmeenergie anlagenintern in einem Wärmespeicher zwischengespeichert und Brauprozessen zugeführt. Damit lässt sich die verfügbare Wärmeenergie zeitlich puffern und gegebenenfalls mit anderen insbesondere ebenfalls regenerativen Wärmequellen der Brauanlage kombinieren.

Vorzugsweise bestehen die der Biomassekonversion zugeführten Braureststoffe zu wenigstens 20 und insbesondere 50 Gew.% aus Treber. Die ansonsten vergleichsweise schlecht zu verwertende Biomasse des Trebers, beispielsweise enthaltenes Lignin, lässt sich durch mehrstufige Biomassekonversion gemeinsam mit Abwasser auf unerwartet effiziente Weise in Biogas mit relativ hohem Methangehalt umsetzen.

Vorzugsweise wird das Biogas mit einem Methangehalt von wenigstens 70%, insbesondere wenigstens 90% gewonnen. Dadurch wird eine Zumischung von Erdgas entbehrlich und ein energieautarker Brauanlagebetrieb begünstigt.

Vorzugsweise werden der Biomassekonversion ferner Hefe und/oder Malzstaub und/oder Heißtrub und/oder Kühltrub zugeführt. Es lassen sich dann alle wesentlichen Reststoffe von Brauprozessen gemeinsam vorbehandeln und effizient in Biogas umsetzen.

Vorzugsweise wird Biomasse maschinell gesteuert aus der zweiten in die erste Konverterstufe und/oder aus der dritten in die zweite Konverterstufe zurückgeführt. Dadurch lassen sich die Vorbehandlungen der Biomasse für deren Fermentation samt der Abscheidung von Wertstoffen an die Zusammensetzung der zugeführten Braureststoffe und/oder eine benötigte Biogasmenge und/oder einen vorgegebenen Methangehalt des Biogases anpassen.

Vorzugsweise wird der Kohlendioxidgehalt im Biogas durch Methanisierung unter Beimengung von Wasserstoff und Verwendung von Methanbakterien aus der Biogasfermentation reduziert. Dadurch sind im Wesentlichen CO₂-neutrale Brauprozesse möglich. Ferner kann der Methangehalt des gewonnenen Biogases zusätzlich erhöht werden.

Vorzugsweise enthält das abgeschiedene Protein-Hydrolysat Rohstoffe zur Verarbeitung in der Lebensmittelindustrie, insbesondere Peptide und Aminosäuren, und/oder das abgeschiedene Mineraldüngemittel enthält wenigstens einen landwirtschaftlichen Düngerstoff, insbesondere Ammoniumsulfat, Diammoniumhydrogenphosphat, Kaliumphosphat, Kalziumsulfat und/oder Magnesiumsulfat.

Durch die beschriebene Wertstoffabscheidung in den Vorbehandlungsstufen können zudem nährstoffhaltige Komponenten der Braureststoffe, beispielsweise Proteine und Kohlenhydrate, beispielsweise für die Herstellung von Nahrungsmitteln stofflich verwertet werden im Unterschied zu einer energetischen Verwertung mittels Biogasfermentation.

Die Brauerei umfasst eine Brauanlage, eine Biomassekonversionsanlage und ein Blockheizkraftwerk, wobei die Biomassekonversionsanlage zur Biomassekonversion von Reststoffen der Brauanlage, insbesondere Treber, umfasst: eine erste Konverterstufe zur Proteolyse unter Abscheidung von Protein-Hydrolysat; ein zweite Konverterstufe zur Versäuerung, und insbesondere zum Ionenaustausch, unter Abscheidung von Mineraldüngemittel; und eine dritte Konverterstufe zur Gewinnung von Biogas für das Blockheizkraftwerk durch Biogasfermentation insbesondere bei gleichzeitiger Aufbereitung von Abwasser zu Prozesswasser.

Die Konverterstufen sind hierzu vorzugsweise separat und/oder zur gegenseitigen Abstimmung maschinell steuerbar.

Die erste und zweite Konverterstufe dienen der Vorbehandlung der zugeführten Braureststoffe für eine effektivere Biogasfermentation in der dritten Konverterstufe und auch der Abscheidung von Wertstoffen. Die abgeschiedenen und ausgeleiteten Mineraldüngemittel würden ansonsten die Ausbeute der Biogasfermentation reduzieren.

Die erste und/oder zweite Konverterstufe kann ferner zum Zellaufschluss ausgebildet sein.

Demgegenüber kann die erste und/oder zweite Konverterstufe aber auch nur zur mechanischen Zerkleinerung der Braureststoffe ausgebildet sein, wobei dabei noch nicht zwangsläufig ein Zellaufschluss bewirkt wird.

Zwischen der ersten und zweiten Konverterstufe und/oder zwischen der zweiten und dritten Konverterstufe kann jeweils wenigstens ein Puffergefäß (Puffertank) geschaltet sein, um zum einen Retentat aus der ersten Konverterstufe für die zweite Konverterstufe zwischenzuspeichern bzw. zum anderen Medium (Sauerwasser) aus der zweiten Konverterstufe für die dritte Konverterstufe zwischenzuspeichern. Das Retentat / Medium (Sauerwasser) kann so zur kontinuierlichen Weiterverarbeitung in der zweiten / dritten Konverterstufe vorgehalten werden, insbesondere auch bei stillstehender erster Konverterstufe.

Somit kann die erste Konverterstufe je nach Bierproduktion diskontinuierlich (chargenweise bzw. als Batch-Prozess) betrieben werden und gegebenenfalls stillstehen oder in Betrieb sein, während die biochemischen Prozesse (basierend auf Mikroorganismen) in der zweiten und dritten Konverterstufe durchgehend aufrechterhalten werden können.

Vorzugsweise umfasst die Brauerei ferner wenigstens einen Gasspeicher zur Zwischenspeicherung gewonnen Biogases und/oder einen Wärmespeicher zur Zwischenspeicherung aus dem Biogas gewonnener Wärme und/oder einen Ladungsspeicher zur Zwischenspeicherung von im Blockheizkraftwerk aus dem Biogas gewonnenem elektrischen Strom. Damit kann das Biogas effizient und bedarfsgerecht für unterschiedliche Brauprozesse eingesetzt werden, wie beispielsweise zum Heizen und Kochen von Brauprodukten in Form von Wärme sowie zum Kühlen und Transportieren von Brauprodukten in Form von Strom.

Vorzugsweise umfasst die Brauerei ferner einen maschinell steuerbaren Gasverteiler zur graduellen Verteilung des gewonnenen Biogases auf einen anlagenintern in einem Gasspeicher zwischenzeitlich zu speichernden ersten Anteil und/oder einen in ein externes Gasnetz einzuspeisenden zweiten Anteil und/oder einen im Blockheizkraftwerk zu verfeuernden dritten Anteil und/oder einen anlagenintern in einem Heizkessel zu verfeuernden vierten Anteil.

Vorzugsweise umfasst die Brauerei eine elektronische Steuereinrichtung zur Steuerung des Gasverteilers in Abhängigkeit von einem betriebsabhängig ermittelten Wärme- und/oder Strombedarf der Brauanlage.

Somit lässt sich das gewonnene Biogas beispielsweise in Abhängigkeit von unterschiedlichen Betriebszuständen der Brauanlage, gegebenenfalls auch bei diesbezüglichem Gasüberschuss, und vorzugsweise automatisch, also ohne Bedienereingriff, optimal verwerten.

Vorzugsweise sind die erste und zweite Konverterstufe derart ausgebildet, dass die Abscheidung von Protein-Hydrolysat, und insbesondere ferner eine vorgeschaltete Abtrennung einer aus dem Zellaufschluss resultierenden proteinhaltigen Fraktion, sowie die Abscheidung von Mineraldüngemittel jeweils separat maschinell gesteuert werden können.

Beispielsweise kann es vorteilhaft sein, Arbeitspunkte der Reststoffverwertung innerhalb gewisser Grenzen zu variieren. So kann zum Beispiel der Anteil des gewonnenen Protein-Hydrolysats reduziert und dadurch die Ausbeute an gewonnenem Biogas gesteigert werden. Zum Beispiel beim Herunterfahren der Produktion, also bei relativ geringem Energiebedarf, könnte dagegen überwiegend oder nur Protein-Hydrolysat gewonnen und die Biogasproduktion entsprechend gedrosselt oder unterbunden werden.

Eine bevorzugte Ausführungsform der Erfindung ist zeichnerisch dargestellt. Es zeigen:
- Fig. 1: ein Schema des Verfahrens mit mehrstufiger Biomassekonversion;
- Fig. 2: ein Schema einer Brauerei mit Medienströmen;
- Fig.3: Prozesse und Stoffströme der Biomassekonversion;
- Fig. 4: eine Ausgestaltung des Verfahrens gemäß Fig. 1 mit zusätzlicher Medienpufferung zwischen den Konverterstufen; und
- Fig. 5: eine Ausgestaltung der Prozesse und Stoffströme gemäß Fig. 3 mit zusätzlicher Aufreinigung des Protein-Hydrolysats.

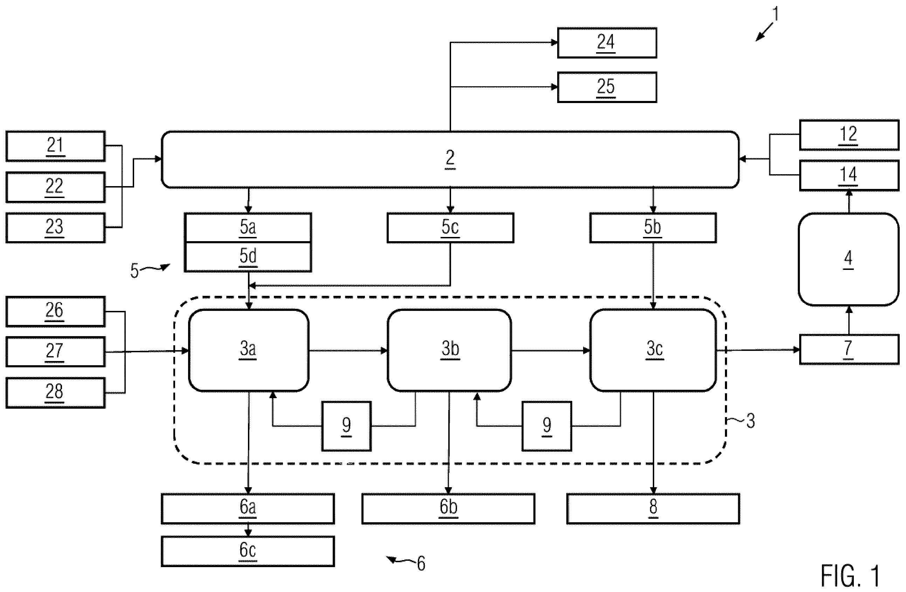

Wie die Fig. 1 erkennen lässt, kann das hier beschriebene Verfahren in einer Brauerei 1 mit einer Brauanlage 2, einer Biomassekonversionsanlage 3 und einem Blockheizkraftwerk 4 durchgeführt werden.

Die Biomassekonversionsanlage 3 umfasst zur Biomassekonversion von Braureststoffen 5 mit Treber 5a und Abwasser 5b und optional ferner mit Hefe 5c und/oder Malzstaub 5d (und/oder Heißtrub und/oder Kühltrub) eine erste Konverterstufe 3a zum optionalen Zellaufschluss und zur Proteolyse unter Abscheidung von Protein-Hydrolysat 6a; eine daran anschließende zweite Konverterstufe 3b zur Versäuerung, und gegebenenfalls ferner zum Ionenaustausch, unter Abscheidung von Mineraldüngemittel 6b; und eine wiederum daran anschließende dritte Konverterstufe 3c zur Gewinnung von Biogas 7 für das Blockheizkraftwerk 4 durch Biogasfermentation vorzugsweise bei gleichzeitiger Aufbereitung des Abwassers 5b zu Prozesswasser 8.

Die erste und zweite Konverterstufe 3a, 3b dienen zum einen der Vorbehandlung wenigstens des Trebers 5a und gegebenenfalls weiterer zugeführter Braureststoffe 5 für deren Biogasfermentation in der dritten Konverterstufe 3c und zum anderen der Abscheidung von Wertstoffen 6 aus den Braureststoffen 5, also des Protein-Hydrolysats 6a (umfassend Peptide und Aminosäuren) und des Mineraldüngemittels 6b (beispielsweise Ammoniumdünger). In der ersten Konverterstufe 3a könnte prinzipiell auch eine nach anfänglichem Zellaufschluss gewonnene Proteinfraktion 6c als Wertstoff 6 gewonnen werden. Dies ist aber nur als zusätzliche Verwertungsoption zu verstehen, beispielsweise je nach Zusammensetzung der Braureststoffe 5.

Die Mineraldüngemittel 6b insgesamt wirken prinzipiell inhibierend auf die Biogasfermentation ein. Somit erhöht die vorgeschaltete Abscheidung und Ausleitung von Mineraldüngemittel 6b die Methanausbeute der hier beschriebenen Biogasfermentation und damit deren Effizienz.

Die Biomassekonversionsanlage 3 umfasst eine (nicht dargestellte) elektronische Steuereinrichtung zur maschinellen Steuerung der einzelnen Konverterstufen 3a, 3b, 3c zum einen unabhängig voneinander und zum anderen in Kombination miteinander bzw. in Abstimmung untereinander.

Somit können die in den Konverterstufen 3a, 3b, 3c ablaufenden chemischen und physikalischen Prozesse, beispielsweise hinsichtlich der Absonderung der Wertstoffe 6 und/oder einer Rückführung von Biomasse 9 und/oder der Erzeugung des Biogases 7 gezielt optimiert werden.

Beispielsweise lassen sich die in der Biomassekonversionsanlage 3 ablaufenden Vorbehandlungen für die anschließende Biogasfermentation und die jeweilige Abscheidung von Wertstoffen 6 an die Zusammensetzung der zugeführten Braureststoffe 5 und/oder die benötigte Menge an Biogas 7 und/oder an einen vorgegebene Methangehalt des Biogases 7 anpassen.

In der Fig. 1 ist als Beispiel eine maschinell gesteuerte Rückführung von Biomasse 9 aus der zweiten Konverterstufe 3b in die erste Konverterstufe 3a und/oder aus der dritten Konverterstufe 3c in die zweite Konverterstufe 3b schematisch angedeutet.

Zu möglichen Prozessvarianten der mehrstufigen Biomassenkonversion in den Konverterstufen 3a, 3b, 3c kann prinzipiell auf die in der DE 10 2016 014 103 B4 beschriebenen Ausführungsformen verwiesen werden.

Gegenüber den dort beschriebenen Verfahren wird die Vorbehandlung in den ersten beiden Konverterstufen 3a, 3b bei dem hier beschriebenen Verfahren allerdings an Braureststoffen 5 mit Treber 5a durchgeführt, der vorzugsweise den größten Gewichtsanteil der insgesamt umgesetzten Braureststoffe 5 ausmacht oder zumindest 20% davon.

Hierfür wird der Biomassekonversionsanlage 3, beispielsweise der dritten Konverterstufe 3c, ferner Abwasser 5b der Brauanlage 2 als Braureststoff 5 zugeführt. Im Treber 5a und im Abwasser 5b enthaltene Biomasse wird somit in derselben Biomassekonversionsanlage 3 regenerativ verwertet, was zudem eine besonders hohe Methanausbeute bewirkt.

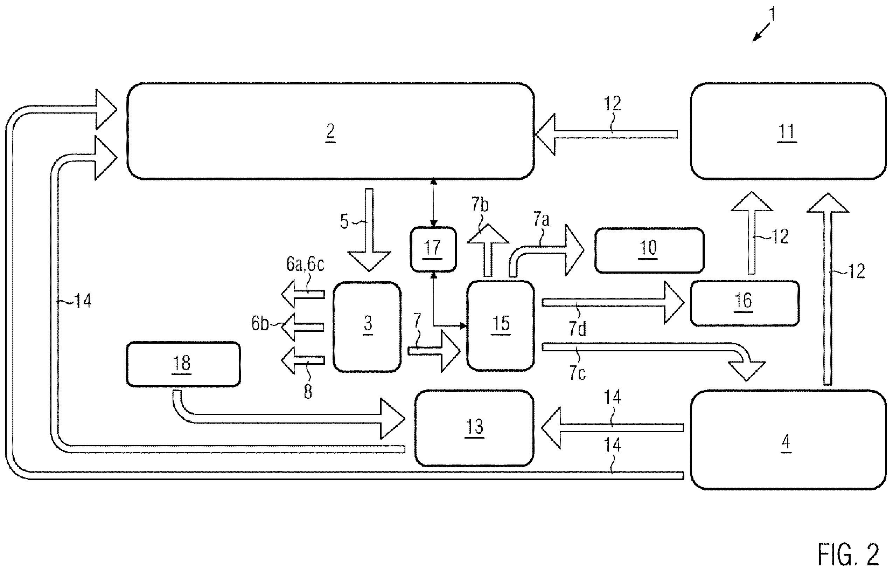

Wie die Fig. 2 erkennen lässt, kann die Brauerei 1 bei einer erweiterten Ausführungsform umfassen: wenigstens einen Gasspeicher 10 zur Zwischenspeicherung gewonnen Biogases 7; und/oder einen Wärmespeicher 11 zur Zwischenspeicherung aus dem Biogas 7 gewonnener Wärme 12, beispielsweise einen Schichtspeicher für Heißwasser als Heizmedium; und/oder einen elektrischen Ladungsspeicher 13 (Akkumulator), der mit im Blockheizkraftwerk 4 aus dem Biogas 7 gewonnenem elektrischen Strom 14 geladen wird und elektrischen Strom 14 bei Strombedarf an die Brauanlage 2 abgibt.

Somit kann das Biogas 7 effizient und bedarfsgerecht für unterschiedliche Brauprozesse in der Brauanlage 2 eingesetzt werden, beispielsweise zum Heizen und Kochen in Form von Wärme 12 sowie zum Kühlen und Transportieren in Form von elektrischem Strom 14.

Überschüssiger elektrischer Strom 14 aus dem Blockheizkraftwerk 4 könnte, gegebenenfalls über den Ladungsspeicher 13, in ein externes Stromnetz (nicht dargestellt) eingespeist und dadurch verwertet werden.

Die Brauerei 1 umfasst vorzugsweise ferner einen maschinell steuerbaren Gasverteiler 15 zur graduellen Verteilung des gewonnen Biogases 7 auf einen anlagenintern im Gasspeicher 10 zwischenzeitlich zu speichernden ersten Anteil 7a des Biogases 7 und/oder einen in ein externes Gasnetz (nicht dargestellt) einzuspeisenden zweiten Anteil 7b des Biogases 7 und/oder einen im Blockheizkraftwerk 4 zu verfeuernden dritten Anteil 7c des Biogases und/oder einen anlagenintern in einem Heizkessel 16 zur Erzeugung von Wärme 12 zu verfeuernden vierten Anteil 7d des Biogases 7.

Der Gasverteiler 15 kann auf prinzipiell bekannte Weise Ventile umfassen und/oder von einer (lediglich schematisch angedeuteten) elektronischen Steuereinrichtung 17 der Brauerei 1 beispielsweise in Abhängigkeit von einem betriebsabhängig ermittelten Wärme- und/oder Strombedarf der Brauanlage 2 gesteuert / geregelt werden.

Mit dem Gasverteiler 15 kann Biogas 7 in Abhängigkeit von unterschiedlichen Betriebszuständen der Brauanlage 2 vorzugsweise automatisch, also ohne Bedienereingriff, geeignet verteilt und folglich optimal verwertet werden. Bei einem Gasüberschuss, also bei einer über den jeweils bestehenden Energiebedarf der Brauanlage 2 hinausgehenden Produktion von Biogas 7, kann beispielsweise dessen erster und/oder zweiter Anteil 7a, 7b erhöht werden, um überschüssiges Biogas 7 vorübergehend zu speichern bzw. durch anlagenexterne Verwendung zu verwerten.

Für den Gasverteiler 15 können nominelle Verteilungsverhältnisse für bestimmte Betriebszustände der Brauanlage 2 im Rahmen der Prozesssteuerung beispielsweise durch entsprechende Programmierung der Steuereinrichtung 17 vorgegeben werden. Beispielsweise könnte der erste Anteil 7a nominell 20% betragen, der zweiter Anteil 7b nominell 0%, der dritte Anteil 7c nominell 70% und der vierte Anteil 7d nominell 10%.

Nominelle Verteilungsverhältnisse für das erzeugte Biogas 7 könnten dann innerhalb geeignet vorgegebener Grenzen automatisch beispielsweise je nach Betriebszustand der Brauanlage 2 und je nach Anforderung für einen praktikablen Betrieb des Blockheizkraftwerks 4 oder auch des Heizkessels 16 und/oder je nach Restkapazität des Gasspeichers 10 angepasst werden. Die Nutzung des erzeugten Biogases 7 lässt sich auf diese Weise besonders flexibel optimieren.

Je nach Kapazität der Biomassekonversionsanlage 3 ist es denkbar, zusätzliche regenerative Energiequellen beispielsweise zum Laden des Ladungsspeichers 13 einzubinden, hier beispielhaft eine Photovoltaik-Solarmoduleinheit 18 zur Stromerzeugung. Obwohl nicht dargestellt, kann alternativ oder zusätzlich zu einem Heizkessel 16 beispielsweise eine Solarthermieanlage zur regenerativen Erzeugung von Prozesswärme eingesetzt werden.

In der Fig. 1 sind zudem prinzipiell bekannte Ausgangsstoffe und Produkte des Bierbrauens in der Brauanlage 2 sowie Hilfsstoffe für die Biomassekonversionsanlage 3 schematisch angedeutet, und zwar Malz 21 (oder andere stärkehaltige Rohstoffe wie beispielsweise Gerste, Mais, Reis), Frischwasser 22, Hopfen 23, Bier 24, Kohlendioxid 25, Säure 26, Lauge 27 und Enzyme 28.

Mit dem beschriebenen Verfahren ist ergänzend oder alternativ zu einem energieautarken Produktionsbetrieb der Brauanlage 2 zudem deren CO₂-neutraler Produktionsbetrieb möglich.

Zusätzlich kann der Kohlendioxidgehalt im Biogas 7 durch Methanisierung unter Beimengung von Wasserstoff und Verwendung von Methanbakterien aus der Biogasfermentation reduziert und dadurch auch der Methangehalt erhöht werden. Auch andere Verfahren stehen zur CO₂-Reduktion am Ende der Biogasfermentation zu Verfügung, siehe beispielsweise die diesbezüglich in der DE 10 2016 014 103 B4 beschriebenen Prozesse.

Die Brauerei 1 kann neben der obligatorischen Brauanlage 2, der Biomassekonversionsanlage 3 und dem Blockheizkraftwerk 4 modular mit einzelnen der beschriebenen Anlagenkomponenten ausgelegt oder gegebenenfalls erweitert werden, also beispielsweise mit einem Gasspeicher 10, einem elektrischen Ladungsspeicher 13 und/oder einem Gasverteiler 15. Je nach regenerativer Kapazität der Biomassekonversionsanlage 3 und der mit den Anlagenkomponenten ermöglichten Flexibilität der Biogasverwendung und/oder Zwischenspeicherung der einzelnen Energieträger kann der externe Energie- und Ressourcenbedarf der Brauanlage 2 bis hin zu energieautarker Betriebsweise durch geeignete modulare Auslegung / Erweiterung der Brauerei 1 gesenkt werden.

Eine entsprechende Anlageauslegung ist beispielsweise auf der Grundlage von Simulationen und Verbrauchsmessungen möglich. Ebenso hat sich in der Praxis dadurch bestätigt, dass die in den Braureststoffen 5 enthaltene Biomasse und daraus zurückgewinnbare Energie ausreicht, um die Brauanlage 2 als Bestandteil der beschriebenen Brauerei 1 energieautark zu versorgen.

Ein prinzipiell angestrebter energieautarker Betrieb der Brauanlage 2 ist auf der Grundlage der mehrstufigen Biomassekonversion in der Biomassekonversionsanlage 3 und der dadurch gesteigerten Rückgewinnungseffizienz prinzipiell auch nur mit einigen der dargestellten Komponenten der Brauerei 1 möglich. Gegebenenfalls kann beispielsweise eine Einspeisung in externe Netze, eine anlageninterne Gasspeicherung, eine Solarstromgewinnung oder dergleichen entbehrlich sein.

Selbst falls eine Nachrüstung bestehender Brauanlagen 2 mit bestimmten Komponenten der hier beschriebenen Brauerei 1 beispielsweise aus baulichen oder wirtschaftlichen Gründen im Einzelfall ausscheiden sollte, so ist dennoch bereits mit der beschriebenen Grundkonfiguration der Brauerei 1 ein gegenüber herkömmlichen Brauereien mit Biogasgewinnung deutlich verringerter Bedarf für externe Energiezufuhr bis hin zum energieautarken Betrieb von Brauanlagen mit vergleichsweise geringem apparativen Aufwand möglich.

Wenn die Rede von Malzstaub ist, so ist hier Staub jeglichem Stärketräger wie beispielsweise Gerste, Reis oder Mais umfasst.

Wenn von Brauprozessen gesprochen wird, so sind auch vor- oder nachbereitende Prozesse wie etwa Reinigungsprozesse umfasst.

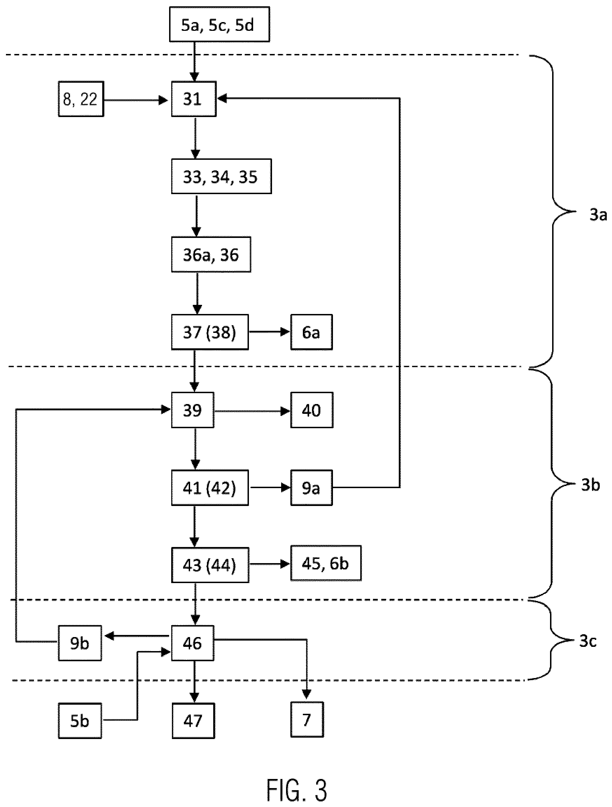

Die Figur 3 zeigt eine Ausführungsform des Verfahrens mit Prozessen und Stoffströmen der Biomassekonversion in der ersten, zweiten und dritten Konverterstufe 3a, 3b und 3c.

Demnach erfolgt in der ersten Konverterstufe 3a zuerst eine Suspendierung 31 der in Form von Treber 5a, Hefe 5c und/oder Malzstaub 5d (und/oder Heißtrub und/oder Kühltrub) zugeführten Braureststoffe 5 mit Frischwasser 22 zu einer Suspension, welche beispielsweise einen Trockenstoffanteil von 10 bis 12 % aufweist. Um den Wasserbedarf zu reduzieren, kann zur Suspendierung anstelle von Frischwasser 22 oder in Ergänzung dazu auch Prozesswasser 8 verwendet werden und/oder Glattwasser, welches im Brauprozess anfällt, und/oder Stapelwasser, welches im Rahmen der Anlagenreinigung mittels einer CIP-Anlage (Cleaning in Place) aufgefangen wird.

Die derart suspendierten Braureststoffe 5 werden in der ersten Konverterstufe 3a ferner einer Dispergierung 33 (Verteilung einer dispersen Phase in einer fluiden Phase) und einer Zerkleinerung 34 unterworfen. Zur Zerkleinerung 34 kann beispielsweise eine Kolloidmühle oder eine Rührwerks-Kugelmühle verwendet werden.

Die Zerkleinerung 34 kann optional bis zu einem Zellaufschluss 35 erfolgen, dieser ist prinzipiell aber auch durch Zugabe von Enzymen und/oder insbesondere gleichzeitige Ultraschallbehandlung möglich oder auf anderweitig chemisch-physikalischem Wege.

Die zerkleinerte Suspension wird in der ersten Konverterstufe 3a anschließend einer Proteolyse 36a unterzogen. Unter der Proteolyse 36a wird hier stets eine enzymatische Hydrolyse 36 von Proteinen verstanden. Der (optionale) Zellaufschluss 35 und die (obligatorische) Proteolyse 36a können in einem gemeinsamen Behälter oder nacheinander in getrennten Behältern durchgeführt werden.

Die Proteolyse 36a findet in einem beheizbaren Behälter statt, wobei die zugeführte Suspension beispielsweise mit einem Rührwerk oder einer Umpumpvorrichtung möglichst homogen gehalten wird. Für einen effizienten Energieeintrag zum Erreichen einer für die eingesetzten Enzyme 28 (siehe Fig. 1) geeigneten Reaktionstemperatur kann eine sogenannte Pillow-Plates-Heizfläche (mit produktseitigen kissenartigen Unebenheiten) oder dergleichen verwendet werden.

Vorrangiges Ziel einer enzymatischen Hydrolyse 36, also der Proteolyse 36a, ist der Abbau von Proteinen in Peptide und Aminosäuren, sodass diese nach der Proteolyse 36a in (chemisch) gelöster Form vorliegen und durch anschließende erste Abtrennung 37 abgeschieden, ausgeleitet und somit getrennt verwertet werden können. Für die Proteolyse 36a können technische Enzyme 28 in Reinform oder als funktionelle Mischung zugegeben werden.

Es können auch weitere technische Enzyme 28 zugesetzt werden, denn die Hydrolyse 36 muss nicht auf eine Proteolyse 36a beschränkt sein. So können beispielsweise lignozellulosische Inhaltsstoffe während der Proteolyse 36a durch Zugabe weiterer technischer Enzyme 28 gespalten werden. Ein solches Vorgehen ist vorteilhaft aber nicht zwingend erforderlich.

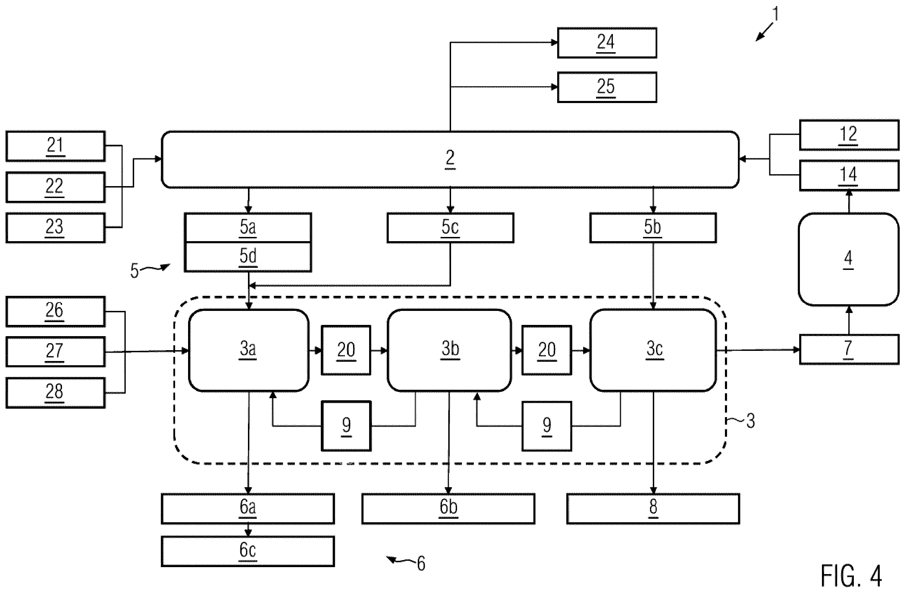

Die erste Abtrennung 37 von Peptiden und Aminosäuren in Form flüssigen Protein-Hydrolysats 6a ist beispielsweise mittels einer ersten Trenneinheit 38 zur Separation, insbesondere Zentrifugalseparation, oder zur Membranfiltration, insbesondere Ultrafiltration, möglich. Es sind jedoch auch andere Trennvorrichtungen, beispielsweise anderweitige Separatoren, Dekanter und/oder Zentrifugen alternativ oder zusätzlich vorgelagert einsetzbar. Das daraus resultierende Retentat kann optional zunächst einem Puffergefäß 20 (in der Figur 4 dargestellt) zugeführt werden. Alternativ kann es geeignet aufkonzentriert, mit beispielsweise 15 bis 25 % Trockenstoffanteil, direkt in die zweite Konverterstufe 3b geleitet werden. Alle Inhaltsstoffe, welche in der Hydrolyse 36 nicht gelöst bzw. infolge der ersten Abtrennung 37 nicht ausgeleitet werden, sind demzufolge im Retentat aufkonzentriert. Die erste Abtrennung 37 ist prozesstechnisch der ersten Konverterstufe 3a zugeordnet. Die dafür verwendete erste Trenneinheit 38 kann räumlich jedoch relativ flexibel im Bereich der ersten Konverterstufe 3a oder zwischen dieser und der zweiten Konverterstufe 3b angeordnet werden.

Durch die erste Abtrennung 37 des Protein-Hydrolysats 6a werden vorzugsweise nur noch 50 bis 80 % des ursprünglichen Stickstoffgehalts (der der ersten Konverterstufe 3a zugeführten Braureststoffe 5) der zweiten Konverterstufe 3b zur dortigen anaeroben Hydrolyse / Versäuerung 39 zugeführt. Diese erfolgt in einem der Biogasgewinnung vorgelagerten Fermentationsprozess mit säurebildenden Mikroorganismen, bei dem Sauergas 40 im Wesentlichen bestehend aus CO₂, H₂ und H₂S entsteht. Dieses Sauergas 40 kann nach geeigneter Reinigung zur energetischen Verwertung, beispielsweise im Blockheizkraftwerk 4, verwendet werden.

Die anaerobe Hydrolyse / Versäuerung 39 wird in einem (durch Heizen und/oder Kühlen) temperierbaren Rührbehälter (nicht dargestellt) der zweiten Konverterstufe 3b durchgeführt. Das gesamte Fermentationsfluid läuft zum Zwecke einer zweiten Abtrennung 41 kontinuierlich im Kreislauf durch eine geeignete Trennvorrichtung, insbesondere eine zweite Trenneinheit 42 zur Membranfiltration, insbesondere Ultrafiltration. Es sind jedoch auch andere Trennvorrichtungen wie beispielhaft ein Separator alternativ oder zusätzlich vorgelagert einsetzbar. Das Fermentationsfluid kann auch in einem Batch-Prozess einer zweiten Abtrennung 41 zugeführt werden.

Die durch die Versäuerung 39 in wässrige Lösung gegangenen Bestandteile wie beispielsweise Ammonium-, Phosphat- und Metallionen werden durch die zweite Abtrennung 41 von nicht hydrolysierter Feststofffracht 9a kontinuierlich getrennt und einem Ionenaustausch 43 in einem lonentauscher 44 zugeführt. Das daraus resultierende Regenerat ist eine Nähr- und Düngerlösung 45, welche das Mineraldüngemittel 6b enthält bzw. zu diesem aufbereitet werden kann. Das heißt, das Mineraldüngemittel 6b liegt in der Nähr- und Düngerlösung 45 in chemisch gelöster Form vor, also flüssig, nicht als Suspension. Beispielsweise wird der Ionentauscher 44 mit Lauge und Säure regeneriert, das zugehörige Regenerat ist die Düngerlösung 45.

Die Nähr- und Düngerlösung 45 bzw. das Mineraldüngemittel 6b wird somit aus der flüssigen Phase der anaeroben Versäuerung 39 (einer Vorfermentation in der zweiten Konverterstufe 3b) und nicht erst aus Gärreste 9b der Methan/Biogas-Fermentation 46 (der Hauptfermentation in der dritten Konverterstufe 3c) gewonnen.

Eine Abtrennung speziell von Ammoniumionen bereits vor der Methan/Biogas-Fermentation 46 bringt enorme Vorteile für den Wirkungsgrad der dritten Konverterstufe 3c, da Ammoniumionen verschiedene Bakterien in der dritten Konverterstufe 3c hemmen, wodurch die Methan/BiogasFermentation 46 stark behindert würde.

Aufgrund des erfindungsgemäßen Prozessablaufs mit vorgezogener Nährstoffabspaltung in Form von Protein-Hydrolysat 6a sowie Nähr-und Düngerlösung 45 bzw. Mineraldüngemittel 6b infolge der Proteolyse 36a in der ersten Konverterstufe 3a und der anaeroben Versäuerung 39 in der zweiten Konverterstufe 3b, also vor der Methan-Fermentation 46, kann die dritte Konverterstufe 3c etwa um das Vier- bis Sechsfache kleiner ausgelegt werden als bei bekannten Verfahren mit dortiger bzw. nachgelagerter Nährstoffabspaltung.

Die erste Abtrennung 37 in der ersten Konverterstufe 3a, die zweite Abtrennung 41 in der zweiten Konverterstufe 3b und der Ionenaustausch 43 in der zweiten Konverterstufe 3b jeweils vor der Methan/Biogas-Fermentation 46 zur Gewinnung des Biogases 7 verbessern die Leistung der Methan produzierenden Bakterien, was nicht nur kleineren Anlagen ermöglicht, sondern auch zu höheren Methankonzentrationen führt. Letztere können durch die Zugabe erzeugten Sauergases 40 zur Methan/Biogas-Fermentation 46 (nicht dargestellt) zusätzlich erhöht werden, weil im Sauergas 40 enthaltener Wasserstoff im Biogas 7 mit Kohlendioxid zu Methan umgesetzt wird.

Die aus der Versäuerung 39 und zweiten Abtrennung 41 resultierende Feststofffracht 9a kann als Biomasse 9 (Fig. 1) in die erste Konverterstufe 3a zurückgeführt werden.

Aus dem anschließenden Ionenaustausch 43 resultierendes Sauerwasser wird in die dritte Konverterstufe 3c geleitet, um dort das Biogas 7 mit Methan-Bakterien zu erzeugen. Der dritten Konverterstufe 3c wird hierfür ferner Abwasser 5b als Bestandteil der Braureststoffe 5 zugeführt.

Die Gärreste 9b aus der dritten Konverterstufe 3c können als Biomasse 9 (Fig. 1) vorzugsweise wieder der zweiten Konverterstufe 3b zugeführt werden.

Durch eine (im laufenden Prozess vorzugsweise ständige) Rückführung von Biomasse 9 aus der dritten Konverterstufe 3c in die zweite Konverterstufe 3b und aus der zweiten Konverterstufe 3b in die erste Konverterstufe 3a wird erreicht, dass kein oder zumindest weniger aus den Braureststoffen 5 resultierender Schlamm anderweitig entsorgt werden muss.

Nach der Methan/Biogas-Fermentation 46 verbleibendes Rest-Abwasser 47 kann aus der dritten Konverterstufe 3c beispielsweise einer Kläranlage zugeführt und/oder als Prozesswasser 8 (Fig. 2) verwendet bzw. zu solchem aufbereitet werden. Hierfür wird das Rest-Abwasser 47 beispielsweise einer klassischen aeroben Abwasserreinigung zugeführt und anschließend entkeimt.

Die beschriebene Aufbereitung von Abwasser 5b umfasst zumindest eine anaerobe Abwasserbehandlung, bei welcher im Abwasser 5b enthaltene organische Substanzen in das Biogas 7 umgewandelt werden. Auf diese anaerobe Abwasserbehandlung kann eine aerobe Abwasserbehandlung und/oder eine Entkeimung folgen. Die aerobe Abwasserbehandlung und/oder die Entkeimung kann ebenfalls Teil der Brauerei 1 sein.

Die Figur 4 zeigt eine vorteilhafte Ausgestaltung des Verfahrens gemäß Figur 1 mit zusätzlicher optionaler Zwischenspeicherung (Pufferung) von aus den Braureststoffen 5 resultierendem Retentat in einem Puffergefäß 20 zwischen der ersten und zweiten Konverterstufe 3a, 3b und von aus dem Retentat resultierendem Medium (Sauerwasser) in einem Puffergefäß 20 zwischen der zweiten und dritten Konverterstufe 3b, 3c. Vor allem die Pufferung zwischen erster und zweiter Konverterstufe 3a, 3b dient der Aufrechterhaltung geeigneter Prozessbedingungen für Mikroorganismen in der zweiten und dritten Konverterstufe 3b, 3c bei diskontinuierlichem Betrieb der vorgelagerten ersten Konverterstufe 3a, der die Braureststoffe 5 produktionsbedingt in der Regel ungleichmäßig zugeführt werden müssen. Die Medienpufferung zwischen der zweiten und dritten Konverterstufe 3b, 3c unterstützt eine solche Betriebsweise der Biomassekonversionsanlage 3, ist gegebenenfalls aber auch entbehrlich.

Zu den übrigen Bestandteilen der Figur 4 wird auf die Erläuterungen zur Figur 1 verwiesen.

Da die erste Konverterstufe 3a bevorzugt diskontinuierlich (als Batch-Prozess) und die zweite Konverterstufe 3b bevorzugt kontinuierlich arbeiten, ist ein Puffergefäß 20 zumindest zwischen der ersten Konverterstufe 3a und der zweiten Konverterstufe 3b vorteilhaft. Somit kann die zweite Konverterstufe 3b (und die dritte Konverterstufe 3c) ohne Unterbrechung (beispielsweise über ein Wochenende ohne Bierproduktion) betrieben werden und muss lediglich für Reinigungs- und Wartungszwecke abgeschaltet werden. Es können Puffergefäße (Puffertanks oder dergleichen) 20 zwischen der ersten Konverterstufe 3a und der zweiten Konverterstufe 3b und/oder zwischen der zweiten Konverterstufe 3b und der dritten Konverterstufe 3c vorhanden sein.

Da die Prozesse in der zweiten Konverterstufe 3b und dritten Konverterstufe 3c jeweils auf Mikroorganismen basieren, sollten diese Prozesse möglichst kontinuierlich aufrechterhalten werden. Deshalb sollte der zweiten Konverterstufe 3b kontinuierlich Retententat aus der ersten Konverterstufe 3a und der dritten Konverterstufe 3c kontinuierlich Medium (Sauerwasser) aus der zweiten Konverterstufe 3b zugeführt werden.

Demgegenüber eignen sich die enzymatischen Prozesse in der ersten Konverterstufe 3a gut für einen diskontinuierlichen Betrieb (Batch-Prozess). Da mit der Brauanlage 2 in der Regel nicht rund um die Uhr und an jedem Tag Bier hergestellt wird und somit nicht gewährleistet werden kann, dass die Braureststoffe 5 kontinuierlich anfallen oder vorrätig sind, ist das Zwischenschalten eines Puffergefäßes 20 vor allem zwischen der ersten und zweiten Konverterstufe 3a, 3b vorteilhaft. Die vorzugsweise kontinuierlichen Prozesse in der zweiten und dritten Konverterstufe 3b, 3c könnten auch so aufeinander abgestimmt werden, dass eine Medienpufferung dazwischen entbehrlich ist. Dennoch kann diese zur flexibleren bzw. stabileren Prozessführung beitragen.

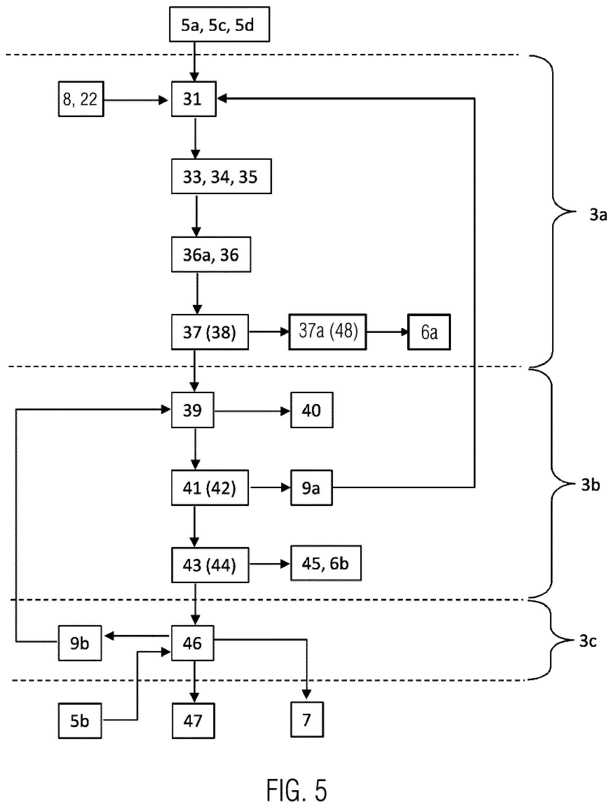

Die Figur 5 zeigt eine optionale Ausgestaltung der Biomassekonversion gemäß Figur 3 mit einer zusätzlichen Aufreinigung 37a des Protein-Hydrolysats 6a. Demnach kann die erste Abtrennung 37 von Peptiden und Aminosäuren in Form flüssigen Protein-Hydrolysats 6a in der ersten Trenneinheit 38 (basierend auf einer Separation, insbesondere Zentrifugalseparation, oder einer Membranfiltration, insbesondere Ultrafiltration) durch zusätzliche Aufreinigung 37a in einer nachgeschalteten Aufreinigungseinheit 48 gegebenenfalls verbessert werden.

Die Aufreinigungseinheit 48 kann beispielsweise eine Einheit zur Proteinfällung und anschließenden Filtration und/oder Zentrifugalseparation ausgefallener Peptide und/oder Aminosäuren sein und/oder eine Einheit zur chromatographischen Trennung.

Hinsichtlich der übrigen Bestandteile der Figur 5 wird auf die Erläuterungen zur Figur 3 verwiesen.

## Patentansprüche

1. Verfahren zum Betreiben einer Brauerei (1), wobei darin anfallende Braureststoffe (5) umfassend Treber (5a) und alkalisches kohlehydrathaltiges Abwasser (5b) einer Biomassekonversion durch Proteolyse (36a) unter Abscheidung von Protein-Hydrolysat (6a) in einer ersten Konverterstufe (3a), durch Versäuerung (39), und insbesondere Ionenaustausch (43), unter Abscheidung von Mineraldüngemittel (6b) in einer zweiten Konverterstufe (3b) und durch Biogasfermentation (46) zur Gewinnung von Biogas (7) bei gleichzeitiger Aufbereitung des Abwassers (5b) in einer dritten Konverterstufe (3c) unterzogen wird, und wobei das Biogas wenigstens anteilig zur Strom- und/oder Wärmeversorgung der Brauerei verfeuert wird.

2. Verfahren nach Anspruch 1, wobei die Biomassekonversion eine mechanische Zerkleinerung (34) der Braureststoffe (5) in der ersten und/oder zweiten Konverterstufe (3a, 3b) umfasst.

3. Verfahren nach Anspruch 1 oder 2, wobei die Biomassekonversion einen Zellaufschluss (35) in der ersten und/oder zweiten Konverterstufe (3a, 3b) umfasst.

4. Verfahren nach Anspruch 1, 2 oder 3, wobei das Protein-Hydrolysat (6a) durch eine erste Abtrennung (37) mittels Separation, insbesondere Zentrifugalseparation, und/oder mittels Membranfiltration, insbesondere Ultrafiltration, abgeschieden wird, und/oder wobei eine das Mineraldüngemittel (6b) enthaltende Nähr-und Düngerlösung (45) nach anaerober Versäuerung (39) durch eine zweite Abtrennung (41) mittels Separation, insbesondere Zentrifugalseparation, und/oder mittels Membranfiltration, insbesondere Ultrafiltration, und durch nachgeschalteten Ionenaustausch (43) abgeschieden wird.

5. Verfahren nach Anspruch 4, wobei die erste Abtrennung (37) eine Aufreinigung (37a) des Protein-Hydrolysats (6a) durch Proteinfällung, durch Abtrennung ausgefallener Peptide und/oder Aminosäuren mittels Filtration und/oder Zentrifugalseparation, und/oder durch chromatographische Trennung umfasst.

6. Verfahren nach wenigstens einem der vorigen Ansprüche, wobei man aus der ersten Konverterstufe (3a) in die zweite Konverterstufe (3b) zu leitendes Retentat und/oder aus der zweiten Konvertstufe (3b) in die dritte Konverterstufe (3c) zu leitendes Sauerwasser jeweils in wenigstens einem Puffergefäß (20) zwischenspeichert, insbesondere bei einem diskontinuierlichen Betrieb der ersten Konverterstufe (3a) und einem im Wesentlichen kontinuierlichen Betrieb der zweiten und/oder dritten Konverterstufe (3b, 3c).

7. Verfahren nach wenigstens einem der vorigen Ansprüche, wobei man einen ersten Anteil (7a) des Biogases (7) im Bereich der Brauerei (1) zwischenspeichert und/oder einen zweiten Anteil (7b) des Biogases in ein externes Gasnetz einspeist und/oder einen dritten Anteil (7c) das Biogases (7) in einem Blockheizkraftwerk (4) verfeuert und/oder einen vierten Anteil (7d) des Biogases in einem Heizkessel (16) anlagenintern verfeuert.

8. Verfahren nach Anspruch 7, wobei man mit dem dritten Anteil (7c) des Biogases (7) erzeugten elektrischen Strom (14) Brauprozessen innerhalb der Brauerei (1) und/oder einem anlageninternen elektrischen Ladungsspeicher (13) zuführt und/oder in ein externes Stromnetz einspeist.

9. Verfahren nach Anspruch 7 oder 8, wobei man aus dem dritten und/oder vierten Anteil (7c, 7d) des Biogases (17) erzeugte Wärme (12) anlagenintern in einem Wärmespeicher (11) zwischengespeichert und Brauprozessen zuführt.

10. Verfahren nach wenigstens einem der vorigen Ansprüche, wobei die der Biomassekonversion zugeführten Braureststoffe (5) zu wenigstens 20 Gew.%, insbesondere zu wenigstens 50 Gew.% aus Treber (5a) bestehen.

11. Verfahren nach wenigstens einem der vorigen Ansprüche, wobei der Biomassekonversion ferner Hefe (5c) und/oder Malzstaub (5d) und/oder Heißtrub und/oder Kühltrub zugeführt werden.

12. Verfahren nach wenigstens einem der vorigen Ansprüche, wobei der Kohlendioxidgehalt im Biogas (7) durch Methanisierung unter Beimengung von Wasserstoff und Verwendung von Methanbakterien aus der Biogasfermentation insbesondere in der dritten Konverterstufe (3c) reduziert wird.

13. Brauerei (1) mit einer Brauanlage (2), einer Biomassekonversionsanlage (3) und einem Blockheizkraftwerk (4), wobei die Biomassekonversionsanlage zur Biomassekonversion von Reststoffen (5) der Brauanlage umfassend Treber (5a) und alkalisches kohlehydrathaltiges Abwasser (5b) umfasst: eine erste Konverterstufe (3a) zur Proteolyse (36a) unter Abscheidung von Protein-Hydrolysat (6a); ein zweite Konverterstufe (3b) zur Versäuerung (39), und insbesondere zum Ionenaustausch (43), unter Abscheidung von Mineraldüngemittel (6b); und eine dritte Konverterstufe (3c) zur Gewinnung von Biogas (7) für das Blockheizkraftwerk (4) durch Biogasfermentation bei gleichzeitiger Aufbereitung des Abwassers (5b).

14. Brauerei nach Anspruch 13, wobei die erste Konverterstufe (3a) und/oder die zweite Konverterstufe (3b) ferner zur mechanischen Zerkleinerung (34) der Braureststoffe (5) ausgebildet ist

15. Brauerei nach Anspruch 13 oder 14, wobei die erste Konverterstufe (3a) und/oder die zweite Konverterstufe (3b) ferner zum Zellaufschluss (35) ausgebildet ist.

16. Brauerei nach Anspruch 13, 14 oder 15, wobei die Biomassekonversionsanlage (3) ferner umfasst: eine erste Trenneinheit (38) zur Abscheidung des Protein-Hydrolysats (6a) mittels Separation, insbesondere Zentrifugalseparation, und/oder mittels Membranfiltration, insbesondere Ultrafiltration; und/oder eine zweite Trenneinheit (42) zur Separation, insbesondere Zentrifugalseparation, und/oder zur Membranfiltration, insbesondere Ultrafiltration, mit nachgeschaltetem Ionentauscher (44) zur Abscheidung einer das Mineraldüngemittel (6b) enthaltenden Nähr-und Düngerlösung (45).

17. Brauerei nach Anspruch 16, wobei der ersten Trenneinheit (38) eine Aufreinigungseinheit (48) zur Aufreinigung (37a) des Protein-Hydrolysats (6a) zugeordnet und insbesondere nachgeschaltet ist, insbesondere in Form einer Einheit zur Proteinfällung und anschließenden Filtration und/oder Zentrifugalseparation ausgefallener Peptide und/oder Aminosäuren und/oder in Form einer Einheit zur chromatographischen Trennung.

18. Brauerei nach wenigstens einem der Ansprüche 13 bis 17, ferner mit wenigstens einem Gasspeicher (10) zur Zwischenspeicherung gewonnen Biogases (7) und/oder einem Wärmespeicher (11) zur Zwischenspeicherung aus dem Biogas gewonnener Wärme (12) und/oder einem elektrischen Ladungsspeicher (13) zur Zwischenspeicherung von im Blockheizkraftwerk (4) aus dem Biogas gewonnenem elektrischen Strom (14).

19. Brauerei nach wenigstens einem der Ansprüche 13 bis 18, ferner mit einem maschinell steuerbaren Gasverteiler (15) zur graduellen Verteilung des gewonnenen Biogases (7) auf einen anlagenintern in einem Gasspeicher (10) zu speichernden ersten Anteil (7a) und/oder einen in ein externes Gasnetz einzuspeisenden zweiten Anteil (7b) und/oder einen im Blockheizkraftwerk (4) zu verfeuernden dritten Anteil (7c) und/oder einen anlagenintern in einem Heizkessel (16) zu verfeuernden vierten Anteil (7d), und ferner mit einer Steuereinrichtung (17) zur Steuerung des Gasverteilers (15) in Abhängigkeit von einem insbesondere betriebsabhängig ermittelten Wärme- und/oder Strombedarf der Brauanlage (2).

20. Brauerei nach wenigstens einem der Ansprüche 13 bis 19, wobei die erste Konverterstufe (3a) bezüglich der Abscheidung von Protein-Hydrolysat (6a), und insbesondere bezüglich einer vorgeschalteten Abtrennung einer aus dem Zellaufschluss resultierenden proteinhaltigen Fraktion (6c), sowie die zweite Konverterstufe (3b) bezüglich der Abscheidung von Mineraldüngemittel (6b) separat maschinell gesteuert werden können.

## Claims

1. A method for operating a brewery (1), wherein brewing residues (5) produced therein, comprising spent grains (5a) and alkaline carbohydrate-containing waste water (5b), undergo biomass conversion by proteolysis (36a) with separation of protein hydrolysate (6a) in a first converter stage (3a), by acidification (39), and in particular ion exchange (43), with separation of mineral fertiliser (6b) in a second converter stage (3b) and by biogas fermentation (46) to obtain biogas (7) with simultaneous treatment of the waste water (5b) in a third converter stage (3c), and wherein the biogas is at least proportionally combusted to supply the brewery with electricity and/or heat.

2. The method according to claim 1, wherein the biomass conversion comprises a mechanical comminution (34) of the brewing residues (5) in the first and/or second converter stage (3a, 3b).

3. The method according to claim 1 or 2, wherein the biomass conversion comprises a cell disruption (35) in the first and/or second converter stage (3a, 3b).

4. The method according to claim 1, 2 or 3, wherein the protein hydrolysate (6a) is separated by a first separation (37) by means of separation, in particular centrifugal separation, and/or by means of membrane filtration, in particular ultrafiltration, and/or wherein a nutrient and fertiliser solution (45) containing the mineral fertiliser (6b) is separated after anaerobic acidification (39) by a second separation (41) by means of separation, in particular centrifugal separation, and/or by means of membrane filtration, in particular ultrafiltration, and by downstream ion exchange (43).

5. The method according to claim 4, wherein the first separation (37) comprises purification (37a) of the protein hydrolysate (6a) by protein precipitation, by separation of precipitated peptides and/or amino acids by filtration and/or centrifugal separation, and/or by chromatographic separation.

6. The method according to at least one of the preceding claims, wherein retentate to be conducted from the first converter stage (3a) into the second converter stage (3b) and/or acidulous water to be conducted from the second converter stage (3b) into the third converter stage (3c) is temporarily stored in at least one buffer vessel (20) in each case, in particular during discontinuous operation of the first converter stage (3a) and substantially continuous operation of the second and/or third converter stage (3b, 3c).

7. The method according to at least one of the preceding claims, wherein a first portion (7a) of the biogas (7) is temporarily stored in the area of the brewery (1) and/or a second portion (7b) of the biogas is fed into an external gas network and/or a third portion (7c) of the biogas (7) is combusted in a combined heat and power plant (4) and/or a fourth portion (7d) of the biogas is combusted in a boiler (16) within the plant.

8. The method according to claim 7, wherein the electric current (14) generated with the third portion (7c) of the biogas (7) is fed to brewing processes within the brewery (1) and/or to an internal electrical charge storage unit (13) and/or fed into an external power grid.

9. The method according to claim 7 or 8, wherein heat (12) generated from the third and/or fourth portion (7c, 7d) of the biogas (17) is temporarily stored within the plant in a heat storage (11) and fed to brewing processes.

10. The method according to at least one of the preceding claims, wherein the brewing residues (5) fed to the biomass conversion consist of at least 20 wt.%, in particular at least 50 wt.% of spent grains (5a).

11. The method according to at least one of the preceding claims, wherein yeast (5c) and/or malt dust (5d) and/or hot sludge and/or cold sludge are further supplied to the biomass conversion.

12. The method according to at least one of the preceding claims, wherein the carbon dioxide content in the biogas (7) is reduced by methanisation with the addition of hydrogen and the use of methane bacteria from the biogas fermentation, in particular in the third converter stage (3c).

13. A brewery (1) with a brewing plant (2), a biomass conversion plant (3) and a combined heat and power plant (4), wherein the biomass conversion plant for biomass conversion of residues (5) of the brewing plant comprising spent grains (5a) and alkaline carbohydrate-containing waste water (5b) comprises: a first converter stage (3a) for proteolysis (36a) with separation of protein hydrolysate (6a); a second converter stage (3b) for acidification (39), and in particular for ion exchange (43), with separation of mineral fertiliser (6b); and a third converter stage (3c) for obtaining biogas (7) for the combined heat and power plant (4) by biogas fermentation with simultaneous treatment of the waste water (5b).

14. Brewery according to claim 13, wherein the first converter stage (3a) and/or the second converter stage (3b) is further configured for mechanical comminution (34) of the brewing residues (5).

15. The brewery according to claim 13 or 14, wherein the first converter stage (3a) and/or the second converter stage (3b) is further configured for cell disruption (35).

16. The brewery according to claim 13, 14 or 15, wherein the biomass conversion plant (3) further comprises:
a first separation unit (38) for separating the protein hydrolysate (6a) by means of separation, in particular centrifugal separation, and/or by means of membrane filtration, in particular ultrafiltration; and/or
a second separation unit (42) for separation, in particular centrifugal separation, and/or for membrane filtration, in particular ultrafiltration, with a downstream ion exchanger (44) for separating a nutrient and fertiliser solution (45) containing the mineral fertiliser (6b).

17. The brewery according to claim 16, wherein a purification unit (48) for purifying (37a) the protein hydrolysate (6a) is associated with the first separation unit (38) and in particular connected downstream, in particular in the form of a unit for protein precipitation and subsequent filtration and/or centrifugal separation of precipitated peptides and/or amino acids and/or in the form of a unit for chromatographic separation.

18. The brewery according to at least one of claims 13 to 17, further comprising at least one gas storage (10) for the intermediate storage of biogas (7) obtained and/or a heat storage tank (11) for the intermediate storage of heat (12) obtained from the biogas and/or an electrical charge storage (13) for the intermediate storage of electrical current (14) obtained from the biogas in the combined heat and power plant (4).

19. The brewery according to at least one of claims 13 to 18, further comprising a mechanically controllable gas distributor (15) for the gradual distribution of the biogas (7) obtained to a first portion (7a) to be stored internally in a gas storage (10) and/or a second portion (7b) to be fed into an external gas network and/or a third portion (7c) to be burnt in the combined heat and power plant (4) and/or a fourth portion (7d) to be burnt internally in a boiler (16), and furthermore with a control device (17) for controlling the gas distributor (15) as a function of a heat and/or electricity requirement of the brewing plant (2) determined in particular as a function of operation.

20. The brewery according to at least one of claims 13 to 19, wherein the first converter stage (3a) can be controlled separately by machine with respect to the separation of protein hydrolysate (6a), and in particular with respect to an upstream separation of a protein-containing fraction (6c) resulting from the cell disruption, and the second converter stage (3b) can be controlled separately by machine with respect to the separation of mineral fertiliser (6b).

## Revendications

1. Procédé d'exploitation d'une brasserie (1), dans lequel des résidus de brassage (5), produits sur place et comprenant des drêches (5a) et des eaux usées alcalines contenant des hydrates de carbone (5b) sont soumis à une conversion de biomasse par protéolyse (36a) avec précipitation d'un hydrolysat de protéines (6a) dans un premier étage de convertisseur (3a), par acidification (39), et en particulier par échange d'ions (43) avec précipitation d'engrais minéral (6b) dans un deuxième étage de convertisseur (3b), et par fermentation de biogaz (46) permettant l'obtention de biogaz (7) avec traitement simultané des eaux usées (5b) dans un troisième étage de convertisseur (3c), et dans lequel le biogaz est au moins en partie consommé pour fournir de l'électricité et/ou de la chaleur à la brasserie.

2. Procédé selon la revendication 1, dans lequel la conversion de biomasse comprend un broyage mécanique (34) des résidus de brassage (5) dans le premier et/ou le deuxième étage de convertisseur (3a, 3b).

3. Procédé selon la revendication 1 ou 2, dans lequel la conversion de biomasse comprend une digestion cellulaire (35) dans le premier et/ou le deuxième étage de convertisseur (3a, 3b).

4. Procédé selon la revendication 1, 2 ou 3, dans lequel l'hydrolysat de protéines (6a) est précipité grâce à une première dissociation (37) par séparation, en particulier par séparation centrifuge, et/ou par filtration sur membrane, en particulier par ultrafiltration, et/ou dans lequel une solution nutritive et fertilisante (45) contenant l'engrais minéral (6b) est précipitée, après acidification anaérobie (39), grâce à une deuxième dissociation (41) par séparation, en particulier par séparation centrifuge, et/ou par filtration sur membrane, en particulier par ultrafiltration, et par échange d'ions (43) raccordé en aval.

5. Procédé selon la revendication 4, dans lequel la première dissociation (37) comprend une purification (37a) de l'hydrolysat de protéines (6a) grâce à une précipitation de protéines, grâce à une dissociation, par filtration et/ou séparation centrifuge, des peptides et/ou acides aminés précipités, et/ou par séparation chromatographique.

6. Procédé selon au moins l'une quelconque des revendications précédentes, dans lequel on stocke temporairement, respectivement dans au moins un récipient tampon (20), du rétentat à acheminer du premier étage de convertisseur (3a) jusque dans le deuxième étage de convertisseur (3b) et/ou de l'eau acide à acheminer du deuxième étage de convertisseur (3b) jusque dans le troisième étage de convertisseur (3c), en particulier dans le cas d'une exploitation discontinue du premier étage de convertisseur (3a) et d'une exploitation essentiellement continue du deuxième et/ou du troisième étage de convertisseur (3b, 3c).

7. Procédé selon au moins l'une quelconque des revendications précédentes, dans lequel une première fraction (7a) du biogaz (7) est stockée temporairement dans la zone de la brasserie (1) et/ou une deuxième fraction (7b) du biogaz est injectée dans un réseau de gaz externe et/ou une troisième fraction (7c) du biogaz (7) est consommée dans une centrale de cogénération (4) et/ou une quatrième fraction (7d) du biogaz est consommée en interne dans une chaudière de chauffage (16).

8. Procédé selon la revendication 7, dans lequel le courant électrique (14) généré avec la troisième fraction (7c) du biogaz (7) est alimenté vers des procédés de brassage à l'intérieur de la brasserie (1) et/ou vers un accumulateur de charge électrique interne à l'installation (13) et/ou est injecté dans un réseau électrique externe.

9. Procédé selon la revendication 7 ou 8, dans lequel la chaleur (12) générée à partir de la troisième et/ou de la quatrième fraction (7c, 7d) du biogaz (17) est stockée temporairement en interne dans un accumulateur de chaleur (11) et alimentée vers des procédés de brassage.

10. Procédé selon au moins l'une quelconque des revendications précédentes, dans lequel les résidus de brassage (5) acheminés vers la conversion de biomasse sont constitués d'au moins 20 % en poids, en particulier d'au moins 50 % en poids de drêches (5a).

11. Procédé selon au moins l'une quelconque des revendications précédentes, dans lequel de la levure (5c) et/ou de la poussière de malt (5d) et/ou des lies chaudes et/ou des lies de refroidissement sont en outre acheminées vers la conversion de biomasse.

12. Procédé selon au moins l'une quelconque des revendications précédentes, dans lequel la teneur en dioxyde de carbone dans le biogaz (7) est réduite par méthanisation avec addition d'hydrogène et utilisation de bactéries méthanogènes provenant de la fermentation de biogaz, en particulier dans le troisième étage de convertisseur (3c).

13. Brasserie (1) comprenant une installation de brassage (2), une installation de conversion de biomasse (3) et une centrale de cogénération (4), dans laquelle l'installation de conversion de biomasse permettant la conversion de biomasse de résidus (5) de l'installation de brassage comprenant des drêches (5a) et des eaux usées alcalines contenant des hydrates de carbone (5b) comprend : un premier étage de convertisseur (3a) permettant la protéolyse (36a) avec précipitation de l'hydrolysat de protéines (6a) ; un deuxième étage de convertisseur (3b) permettant l'acidification (39), et en particulier permettant l'échange d'ions (43) avec précipitation d'engrais minéral (6b) ; et un troisième étage de convertisseur (3c) permettant l'obtention de biogaz (7) pour la centrale de cogénération (4) par fermentation de biogaz avec traitement simultané des eaux usées (5b).

14. Brasserie selon la revendication 13, dans laquelle le premier étage de convertisseur (3a) et/ou le deuxième étage de convertisseur (3b) est/sont en outre conçu(s) pour le broyage mécanique (34) des résidus de brassage (5).

15. Brasserie selon la revendication 13 ou 14, dans laquelle le premier étage de conversion (3a) et/ou le second étage de conversion (3b) est/sont en outre conçu(s) pour la digestion cellulaire (35).

16. Brasserie selon la revendication 13, 14 ou 15, dans laquelle l'installation de conversion de biomasse (3) comprend en outre : une première unité de séparation (38) permettant la précipitation de l'hydrolysat de protéines (6a) par séparation, en particulier par séparation centrifuge, et/ou par filtration sur membrane, en particulier par ultrafiltration ; et/ou une deuxième unité de séparation (42) permettant la séparation, en particulier la séparation centrifuge, et/ou la filtration sur membrane, en particulier l'ultrafiltration, avec échangeur d'ions (44) raccordé en aval permettant de précipiter une solution nutritive et fertilisante (45) contenant l'engrais minéral (6b).

17. Brasserie selon la revendication 16, dans laquelle une unité de purification (48) permettant la purification (37a) de l'hydrolysat de protéines (6a) est associée à la première unité de séparation (38) et est en particulier raccordé en aval, en particulier sous la forme d'une unité permettant la précipitation de protéines et la filtration ultérieure et/ou la séparation centrifuge de peptides et/ou d'acides aminés précipités et/ou sous la forme d'une unité permettant la séparation chromatographique.

18. Brasserie selon au moins l'une quelconque des revendications 13 à 17, comprenant en outre au moins un accumulateur de gaz (10) permettant le stockage intermédiaire du biogaz (7) obtenu et/ou un accumulateur de chaleur (11) permettant le stockage intermédiaire de la chaleur (12) obtenue à partir du biogaz et/ou un accumulateur de charge électrique (13) permettant le stockage intermédiaire de l'électricité (14) obtenue à partir du biogaz dans la centrale de cogénération (4).

19. Brasserie selon au moins l'une quelconque des revendications 13 à 18, comprenant en outre un distributeur de gaz à commande mécanique (15) permettant la distribution progressive du biogaz (7) obtenu sur une première fraction (7a) à stocker en interne dans un réservoir de gaz (10) et/ou une deuxième fraction (7b) à injecter dans un réseau de gaz externe et/ou une troisième fraction (7c) à consommer dans la centrale de cogénération (4) et/ou une quatrième fraction (7d) à consommer en interne dans une chaudière de chauffage (16), et comprenant en outre un dispositif de commande (17) permettant de commander le distributeur de gaz (15) en fonction d'une demande de chaleur et/ou d'électricité de l'installation de brassage (2) déterminée en particulier en fonction du fonctionnement.

20. Brasserie selon au moins l'une quelconque des revendications 13 à 19, dans laquelle le premier étage de convertisseur (3a) peut être commandé mécaniquement de manière séparée en ce qui concerne la précipitation de l'hydrolysat de protéines (6a), et en particulier en ce qui concerne la dissociation en amont d'une fraction (6c) contenant des protéines et résultant de la digestion cellulaire, et le deuxième étage de convertisseur (3b) peut être commandé mécaniquement de manière séparée en ce qui concerne la précipitation de l'engrais minéral (6b).
